# EUROPÄISCHE PATENTANMELDUNG

(11) **EP 1 710 567 A1**
(43) Veröffentlichungstag der Anmeldung: **11.10.2006**
(21) Anmeldenummer: 05102787.8
(22) Anmeldetag: 08.04.2005
(51) Int. Cl.: G01N 27/403

(54) **Abwasseranalyse-Sensorkartusche**

(71) Anmelder: Hach Lange GmbH, 14163 Berlin (DE)
(72) Erfinder: Uthemann, Rolf, 50735, Köln (DE); Golitz, Andreas, 47445, Moers (DE); Woodward, John R., 80550, Windsor (US)
(74) Vertreter: Eberlein, Jasper

(57) **Zusammenfassung**

Die erfindungsgemäße Abwasseranalyse-Sensorkartusche (10) ist für den einmaligen Gebrauch bestimmt. Sie weist ein flüssigkeitsdichtes Gehäuse (20) auf, das mindestens ein elektro-chemisches Sensorelement (30,32) und mindestens ein Referenzelement (56) hält. Ferner enthält das Gehäuse (20) einen Sensorelement-Elektrolyttank (40,42) und einen Referenzelement-Elektrolyttank (44). Hiermit ist auf einfache Weise eine Einmal-Sensorkartusche geschaffen, die bei Defekt eines Sensorelementes oder Referenzelementes, oder bei leerem Sensorelement-Elektrolyttank oder Referenzelement-Elektrolyttank vollständig ausgewechselt wird.

## Beschreibung

Die Erfindung bezieht sich auf eine Abwasseranalyse-Sensorkartusche für den einmaligen Gebrauch.

Sensoren für die Analyse von Abwasser in Kläranlagen u.ä. sind schon aufgrund der unwirtlichen Messumgebung, nämlich Abwasser, relativ anfällig für Störungen. Ferner ist für elektro-chemische Sensorelemente bzw. für Referenzelemente jeweils ein Vorrat an Elektrolyt in einem Elektrolyttank vorzusehen. Bei Ausfall des Sensorelementes oder des Referenzelementes, oder aber bei einem leeren Elektrolyttank, muss das betreffende Sensor- bzw. Referenzelement ausgetauscht bzw. der betreffende Elektrolyttank mit Elektrolyt aufgefüllt werden. Die Feststellung, welches Sensorelement bzw. Referenzelement ausgetauscht werden muss, ist bei Fehlfunktionen häufig nicht einfach zu treffen. Das Austauschen einzelner Sensorelemente bzw. das Auffüllen des Elektrolyttanks ist umständlich, zeitaufwändig und kostenintensiv.

Aufgabe der Erfindung ist es demgegenüber, die Reparatur und Wartung einer Abwasseranalyse-Sensorvorrichtung zu vereinfachen.

Diese Aufgabe wird erfindungsgemäß gelöst mit den Merkmalen des Patentanspruches 1.

Gemäß der Erfindung ist eine Abwasseranalyse-Sensorkartusche für den einmaligen Gebrauch vorgesehen. Die Sensorkartusche weist hierzu ein einstückiges flüssigkeitsdichtes Gehäuse auf, das bevorzugt aus Kunststoff gefertigt ist. Das Gehäuse enthält mindestens ein elektro-chemisches Sensorelement und mindestens ein Referenzelement. Das Sensorelement und das Referenzelement werden von der Umfangswand des Gehäuses gehalten. Ferner enthält das Gehäuse einen Sensorelement-Elektrolyttank und einen Referenzelement-Elektrolyttank, in denen sich das für das Sensorelement bzw. das Referenzelement benötigte Elektrolyt befindet. Sobald eines der Elemente defekt ist, oder einer der Elektrolyttanks leer ist, wird die gesamte Sensorkartusche ausgetauscht gegen eine neue Sensorkartusche, die vollständig gefüllte Elektrolyttanks aufweist und ein neues Sensorelement und Referenzelement enthält. Eine Austauschbarkeit des Sensorelementes oder des Referenzelementes oder eine Auffüllbarkeit eines Elektrolyttanks ist nicht vorgesehen.

Die umständliche Suche nach dem defekten Element bzw. der umständliche Austausch einzelner Elemente entfällt. Auch das umständliche Auffüllen eines Elektrolyttanks entfällt. Die Lebensdauer des Sensorelementes und des Referenzelementes kann u.U. auf das Volumen des Elektrolyttanks abgestimmt werden. Das Sensorelement und das Referenzelement müssen nicht länger halten, als der Vorrat in den Elektrolyttanks ausreicht. Je nach Elementart steckt hierin ein weiteres Einsparpotenzial.

Das elektro-chemische Sensorelement kann beispielsweise ein Ammonium-Sensorelement und/oder ein Kalium-Sensorelement zur Kompensation der Querempfindlichkeit des Ammonium-Sensorelementes sein.

Das Referenzelement kann als pH-Sonde ausgebildet sein, die zusammen mit dem Referenzelement-Elektrolyt und einem Stromschlüssel-Element eine Referenzanordnung bildet.

Die Sensorkartusche kann in großen Stückzahlen hergestellt werden, so dass die Herstellungskosten relativ gering sind. Da zum Austausch des Sensorelementes und des Referenzelementes und zum Auffüllen der Elektrolyttanks nur noch wenige Handgriffe erforderlich sind, sind die Wartungs- und Instandhaltungskosten für eine Abwasseranalyse-Anlage, in der die Sensorkartusche zum Einsatz kommt, niedriger als für herkömmliche Abwasseranalyse-Anlagen.

Vorzugsweise bildet das einstückige Gehäuse die Elektrolyttanks. Das Gehäuse weist innenseitig eine komplexe Struktur auf, die mehrere geschlossene oder verschließbare Räume zur Verfügung stellt. In dem Gehäuse lassen sich auf diese Weise praktisch ohne Mehrkosten mehrere Elektrolyttanks bilden. Derartige Strukturen lassen sich problemlos und preiswert im Kunststoff-Spritzguss herstellen.

Gemäß einer bevorzugten Ausgestaltung weist das Gehäuse eine elektro-mechanische Kupplungsanordnung auf, mit der es an eine entsprechende elektro-mechanische Kupplungsanordnung einer landseitig befestigten Haltevorrichtung kuppelbar ist. Die Kupplungsanordnung dient zum einen der mechanischen Befestigung der Sensorkartusche an der Haltevorrichtung, beispielsweise einem Halterahmen, der landseitig neben dem Abwasserbecken montiert ist und mit einem entsprechenden Haltearm in das Klärbecken bzw. in das Abwasser hineinragt. Zum anderen dient die Kupplungsanordnung der elektrischen Verbindung des Sensorelementes und des Referenzelementes mit der landseitigen Anlage, in der die elektrischen Signale des Sensorelementes und des Referenzelementes ausgewertet werden.

Gemäß einer bevorzugten Ausgestaltung weist die Kupplungsanordnung ein Dichtelement auf, so dass sie mit der Kupplungsanordnung der landseitig befestigten Haltevorrichtung flüssigkeitsdicht zusammenkuppelbar ist. Der innere Bereich der aneinander gekuppelten Kupplungsanordnungen ist flüssigkeitsdicht isoliert gegenüber der Umgebung, so dass die elektrischen Verbindungen zwischen den beiden Kupplungsanordnungen keiner Feuchtigkeit bzw. Flüssigkeit ausgesetzt sind.

Vorzugsweise trägt das Gehäuse mindestens zwei oder mehr elektro-chemische Sensorelemente. Ferner kann ein Temperatursensor zusätzlich vorgesehen sein. Auch das Stromschlüssel-Element, beispielsweise in Form einer Salzbrücke, kann von dem Gehäuse getragen werden.

Gemäß einer bevorzugten Ausgestaltung bildet die Gehäusewand des Gehäuses jeweils teilweise die Tankwand der Elektrolyttanks. Die Tankwände der Elektrolyttanks und die Gehäusewand des Gehäuses überlappen sich mindestens teilweise. Auf diese Weise kann ein Teil der Wände gemeinsam als Gehäusewand und als Tankwand fungieren. Hierdurch wird im Innenraum des Gehäuses insgesamt mehr Hohlraum gewonnen, der für die Elektrolyttanks genutzt werden kann. Durch die Überlappung der Wände der Elektrolyttanks miteinander und/oder mit der Seitenwand des Gehäuses können die Elektrolyttanks bei unveränderter Gehäusegröße in ihrem Tankvolumen vergrößert werden.

Gemäß einer bevorzugten Ausgestaltung ist das Gehäuse becherförmig ausge-staltet, wobei das bzw. die Sensorelemente und das Stromschlüssel-Element und ggf. der Temperatursensor am Becherboden angeordnet sind. Die Becheröffnung wird durch einen separaten Deckel verschlossen, der mit dem Becher verklebt oder verschweißt oder auf andere Weise flüssigkeitsdicht verbunden ist. Der Becherdeckel weist die elektrischen Kupplungselemente auf.

Im Folgenden wird unter Bezugnahme auf die Zeichnungen ein Ausführungsbeispiel der Erfindung näher erläutert.

Es zeigen:

Figur 1 eine Abwasseranalyse-Anlage mit einer erfindungsgemäßen Abwasseranalyse-Sensorkartusche,

Figur 2 die Abwasseranalyse-Sensorkartusche der Figur 1 im Querschnitt,

Figur 3 die Abwasseranalyse-Sensorkartusche der Figur 1 im Längsschnitt,

Figur 4 die Abwasseranalyse-Sensorkartusche der Figur 1 in perspektivischer Ansicht von unten, und

Figur 5 die Abwasseranalyse-Sensorkartusche der Figuren 1 bis 4 von der Seite der Kupplungsanordnung aus in perspektivischer Ansicht.

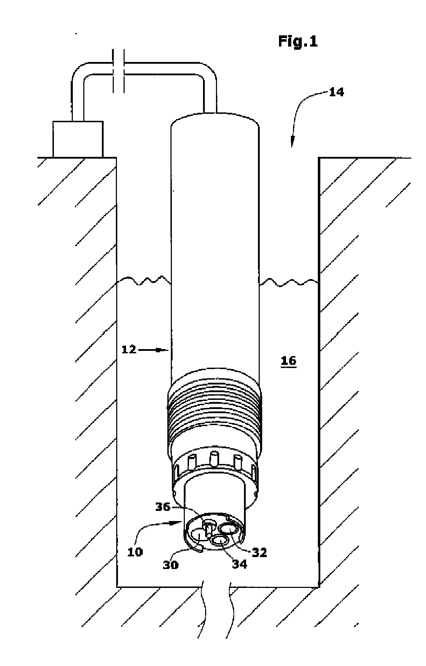

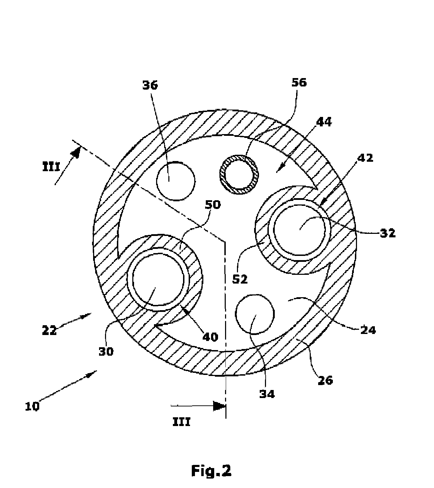

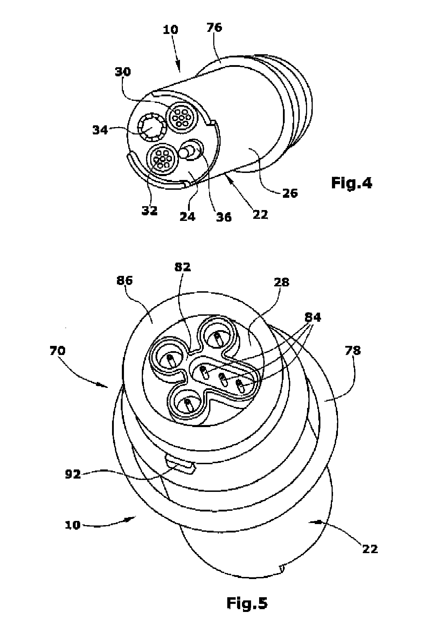

In den Figuren 1 bis 5 ist eine Abwasseranalyse-Sensorkartusche 10 dargestellt, die an einer landseitig befestigten Haltevorrichtung 12 abnehmbar befestigt ist. Die Haltevorrichtung 12 ist zusammen mit der Sensorkartusche 10 in ein Abwasserbecken 14 einer Kläranlage vollständig eingetaucht.

Die Sensorkartusche 10 besteht aus einem einstückigen flüssigkeitsdichten Kunststoff-Gehäuse 20, das wiederum aus einem einstückigen Gehäusebecher 22 mit einer Bodenwand 24, einer Seitenwand 26 sowie einer mit dem Gehäusebecher 22 verklebten oder verschweißten Deckelwand 28 besteht. Der Gehäusebecher 22 ist annähernd zylindrisch ausgebildet.

In der Bodenwand 24 des Bechers 22 sind zwei elektro-chemische Sensorelemente 30,32, ein Stromschlüssel-Element 34 sowie ein Temperatursensor 36 angeordnet. In das Gehäuse 20 bzw. den Becher 22 ragt ein stabförmiges Referenzelement 56 senkrecht von oben hinein.

Das erste elektro-chemische Sensorelement 30 dient der elektro-chemischen Messung von Ammonium, das zweite Sensorelement 32 dient der elektro-chemischen Messung von Kalium zur Kompensation der Querempfindlichkeit der Ammonium-Messung. Das Stromschlüssel-Element 34 ist als sog. Salzbrücke ausgebildet, die als Spannungsüberträger dient, und ein flüssiges oder gelartiges Elektrolyt zwischen zwei Diaphragmen aufweist. Der Temperatursensor 36 dient der Ermittlung der Abwasser-Temperatur.

Den beiden Sensorelementen 30,32 und dem Referenzelement 56 ist jeweils ein Elektrolyttank 40,42,44 zugeordnet, der von Tankwänden 50,52 in dem Gehäuse 20 gebildet wird. An die Gehäuseseitenwand 26 schließen sich die zwei zylindrischen Tankwände 50,52 an, die sich mit der Gehäuseseitenwand 26 teilweise überschneiden, so dass die Gehäuseseitenwand 26 teilweise die Tankwand 50 der beiden nicht weiter ausgekleideten Elektrolyttanks 40,42 bildet. Der verbleibende Innenraum innerhalb des Gehäuses 20 und außerhalb des Elektrolyttanks 40,42 bildet den Elektrolyttank 44 des Referenzelementes 56. Durch die Überschneidungen der Gehäuseseitenwand 26 und der Tankwände 50,52 der Elektrolyttanks 40,42 wird ein größtmögliches Volumen des Referenzelement- Elektrolyttanks 44 ermöglicht.

Das Referenzelement 56 ist als pH-Sonde ausgebildet, die von dem Deckel 28 aus axial Richtung Becher-Bodenwand 24 herabhängt und in das Elektrolyt eingetaucht ist. Das Referenzelement 56, das Elektrolyt in dem Referenzelement-Elektrolyttank 44 und das Stromschlüssel-Element 34 bilden zusammen eine Referenzanordnung, die ein Referenzsignal für die von den beiden Sensorelementen 30,32 gewonnenen Sensorsignale liefert.

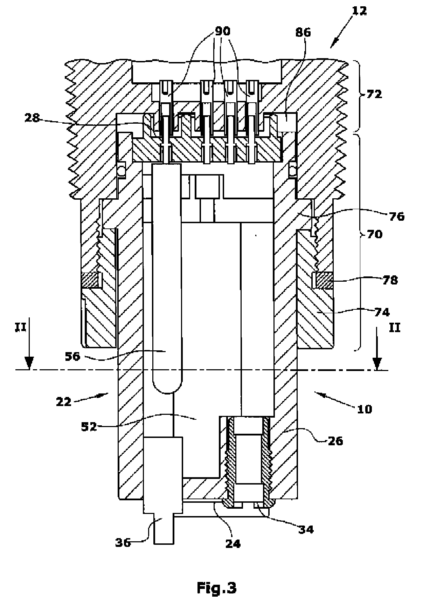

An dem dem Becherboden 24 gegenüberliegenden Längsende des Gehäuses 20 ist eine elektro-mechanische Kupplungsanordnung 70 vorgesehen, die mit einer entsprechenden elektro-mechanischen Kupplungsanordnung 72 der Haltevorrichtung 12 zusammenkuppelbar ist, wie in Figur 3 dargestellt. Die Kupplungsanordnungen 70,72 sind flüssigkeitsdicht miteinander kuppelbar.

Die Kupplungsanordnung 70 der Sensorkartusche 10 weist im Wesentlichen eine Überwurfmutter 74, einen Überwurfmutter-Flansch 76, einen Überwurfmuttern-Dichtungsring 78, einen Radialdichtungsring 80 sowie von Kontaktschutzwänden 82 umgebene Kontaktstifte 84 und einen Abschlussdichtungsring 86 auf.

Die Kontaktstifte 84 sind in entsprechende Kontaktbuchsen 90 der Kupplungsanordnung 72 der Haltevorrichtung 12 eingesteckt. Die Montage bzw. Demontage der Sensorkartusche 10 an bzw. von der Haltevorrichtung 12 erfolgt durch entsprechendes Festschrauben bzw. Abschrauben der Überwurfmutter 74. Ferner sind als Verdrehsicherungen zwei Sicherungsnasen 92 an dem Gehäuse vorgesehen, die in entsprechende Ausnehmungen der Haltevorrichtung 12 eingreifen.

Die Haltevorrichtung 12 weist ein flüssigkeitsdichtes Gehäuse auf, in dem sich die Messelektronik befindet. Durch die Nähe der Messelektronik zu den Sensorelementen und dem Referenzelement können die relativ schwachen Messsignale zuverlässig und genau ausgewertet werden. Die Haltevorrichtung kann an einem Halterohr 13 steif und ortsfest montiert, kann aber auch an einem Kabel hängend aufgehängt sein.

Bei Fehlfunktion, bei rechnerisch ermittelter kurz bevorstehender und/oder signalisierter Erschöpfung eines der Elektrolyte wird die auszutauschende Sensorkartusche 10 nach Lösen der Überwurfmutter 74 abgezogen und eine neue Sensorkartusche 10 durch Ankoppeln und Festschrauben der Überwurfmutter ausgetauscht. Eine zeitaufwändige Fehlersuche, ein Austausch einzelner Elemente oder ein umständliches manuelles Auffüllen der Elektrolyte entfällt.

## Patentansprüche

1. Abwasseranalyse-Sensorkartusche (10) für den einmaligen Gebrauch, mit einem einstückigen flüssigkeitsdichten Gehäuse (20), das mindestens ein elektro-chemisches Sensorelement (30,32) und mindestens ein Referenzelement (56) hält, und das einen Sensorelement-Elektrolyttank (40,42) und einen Referenzelement-Elektrolyttank (44) enthält.

2. Abwasseranalyse-Sensorkartusche (10) nach Anspruch 1, **dadurch gekennzeichnet, dass** das einstückige Gehäuse (20) die Elektrolyttanks (40,42) bildet.

3. Abwasseranalyse-Sensorkartusche (10) nach Anspruch 1 oder 2, **dadurch gekennzeichnet, dass** das Gehäuse (20) eine elektro-mechanische Kupplungsanordnung (70) aufweist, mit der die Sensorkartusche (10) an eine entsprechende elektro-mechanische Kupplungsanordnung (72) einer landseitig befestigten Haltevorrichtung (12) kuppelbar ist.

4. Abwasseranalyse-Sensorkartusche (10) nach Anspruch 3, **dadurch gekennzeichnet, dass** die gehäuseseitige Kupplungsanordnung (70) ein Dichtelement (78,86) aufweist, so dass beide Kupplungsanordnungen (70,72) flüssigkeitsdicht aneinander kuppelbar sind.

5. Abwasseranalyse-Sensorkartusche (10) nach einem der Ansprüche 1 bis 4, **dadurch gekennzeichnet, dass** das Gehäuse (20) mindestens zwei elektro-chemische Sensorelemente (30,32) trägt.

6. Abwasseranalyse-Sensorkartusche (10) nach einem der Ansprüche 1 bis 5, **dadurch gekennzeichnet, dass** das Gehäuse (20) einen Temperatursensor (36) trägt.

7. Abwasseranalyse-Sensorkartusche (10) nach einem der Ansprüche 1 bis 6, **dadurch gekennzeichnet, dass** das Gehäuse ein Stromschlüssel-Element (34) trägt.

8. Abwasseranalyse-Sensorkartusche (10) nach einem der Ansprüche 1 bis 7, **dadurch gekennzeichnet, dass** die Gehäuseseitenwand (26) jeweils teilweise die Wand der Elektrolyttanks (40,42,44) bildet.

9. Abwasseranalyse-Sensorkartusche (10) nach einem der Ansprüche 1 bis 8, **dadurch gekennzeichnet, dass** das Gehäuse (20) becherförmig ist, wobei das Sensorelement (30,32) an der Becher-Bodenwand (24) angeordnet ist.
